(19) Europäisches Patentamt
European Patent Office
Office européen des brevets

(11) Publication number: 0 207 669
A1

(12) # EUROPEAN PATENT APPLICATION

(21) Application number: 86304544.9

(22) Date of filing: 12.06.86

(51) Int. Cl.[4]: C 07 D 331/02
C 07 D 331/04, C 07 D 333/00
C 07 D 335/02, C 07 D 337/00
C 07 D 339/06, C 07 D 327/04

(30) Priority: 14.06.85 GB 8515180

(43) Date of publication of application:
07.01.87 Bulletin 87/2

(84) Designated Contracting States:
BE DE FR GB IT NL

(71) Applicant: BP Chemicals Limited
Belgrave House 76 Buckingham Palace Road
London, SW1W 0SU(GB)

(72) Inventor: Green, Michael James
14 The Sett Yateley
Nr. Camberley Surrey, GU17 7YF(GB)

(74) Representative: Fawcett, Richard Fennelly et al,
BP INTERNATIONAL LIMITED Patents Division Chertsey Road
Sunbury-on-Thames Middlesex, TW16 7LN(GB)

(54) **Catalytic production of sulphur-containing cyclic compounds.**

(57) A sulphur-containing cyclic compound comprising a thioepoxide and/or a cyclic thiocarbonate is produced by reacting carbon disulphide at a temperature in the range from 0 to 100° with an epoxide in the presence as catalyst of an amidine.

EP 0 207 669 A1

CATALYTIC PRODUCTION OF SULPHUR-CONTAINING CYCLIC COMPOUNDS

The present invention relates to a novel process for the production of sulphur-containing cyclic compounds. More specifically, this invention relates to a process for the production of sulphur-containing cyclic compounds by the reaction of carbon disulphide with an epoxide in the presence of a Lewis base catalyst.

Processes for the production of sulphur-containing cyclic compounds are known. Thus USP-A-3,124,596 discloses a process for producing alkylene trithiocarbonates by reacting carbon disulphide at a temperature between 5°C and 250°C and a pressure between 30 and 1500 psi with an alkylene oxide corresponding to the formula:

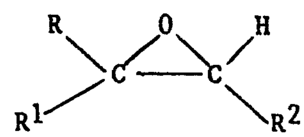

wherein R, $R^1$ and $R^2$ taken separately are selected from the group consisting of hydrogen, aliphatic, alicyclic and aromatic radicals and wherein $R^1$ and $R^2$ taken collectively is an alicyclic group. The process of the invention of USP-A-3,124,596 can be conducted without a catalyst, however for optimum results it reported to be advantageous to include an organic nitrogen-base compound, for example an amine, as catalyst in the reaction medium.

USP-A-3,457,277 describes a process for making vicinal episulphides by reacting a sulphur-donating compound, for example carbon disulphide with an alkylene oxide compound using a novel liquid catalyst system wherein the catalyst system comprises a Lewis

base, for example a basic amine compound or a quaternary ammonium salt, dissolved in an organic solvent. The process of USP-A-3,457,277 is operable over a pressure range from 5 mm Hg up to atmospheric or autogenous pressure and a temperature in the range from about 100°C to about 300°C, preferably from 180 to 220°C.

Vicinal monoepisulphides may be polymerised to form elastomers or moldable thermoplastic polymers. Epoxides, too, are known to polymerise. In the prior art referred to above the problem of side reactions occurring by homo- and copolymerisation of both the epoxide reactant and the sulphur-containing product is acknowledged. Clearly, it would be desirable to use a catalyst system operable under reaction conditions which minimises the previously recognised polymerisation problem.

We have found that amidine catalysts are very effective under mild operating conditions, thereby reducing the risk of forming by-product polymerisation products.

Accordingly, the present invention provides a process for the production of a sulphur-containing cyclic compound comprising a thioepoxide and/or a cyclic thiocarbonate which process comprises reacting carbon disulphide at a temperature in the range from 0 to 100°C with an epoxide in the presence as catalyst of an amidine.

The sulphur-containing cyclic compounds prepared herein are the reaction products of carbon disulphide and epoxides. The epoxides may suitably be compounds containing at least one moiety of the formula:

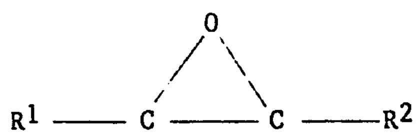

(I)

wherein $R^1$ and $R^2$ taken separately are independently selected from the group consisting of hydrogen, aliphatic, alicyclic and aromatic radicals or $R^1$ and $R^2$ taken collectively is an alicyclic group. Generally, the epoxides useful herein will have from 2 to 12 carbon atoms. Illustrative epoxides include ethylene oxide, propylene oxide, phenyloxirane (styrene oxides), butylene oxide, and the like.

The carbon disulphide reactant can be commercially obtained

from conventional processes such as the reaction of natural gas or petroleum fractions with sulphur or by heating sulphur and charcoal and condensing the carbon disulphide vapours.

The process of the present invention is preferably conducted in the liquid phase in the presence of a solvent. Suitable solvents include alcohol solvents such as $C_1$ to $C_{12}$ aliphatic alcohols and particularly methanol and ethanol or $C_1$ to $C_{12}$ aliphatic thiols, such as butanethiol. Although a wide range of solvents can be employed, the preferred solvents are lower aliphatic alcohols such as methanol and ethanol.

Generally, the molar ratio of the epoxide to carbon disulphide will range from 10:1 to 1:10. However, other molar ratios outside of this range are still operable for the preparation of sulphur-containing cyclic compounds. The solvent concentration may range from 1 to 90% by weight of the total mixture and preferably will constitute from 5 to 50% by weight of the total mixture.

The sulphur-containing cyclic compounds prepared by the process of the present invention are thioepoxides and cyclic thiocarbonates. However, small amounts of other sulphur-containing compounds such as acyclic carbonates are also prepared during the reaction of epoxides with carbon disulphide. The thioepoxides prepared herein will correspond to the reactant epoxide except that the oxygen atom has been replaced with a sulphur atom. For example, if the epoxide reactant is ethylene oxide, the resulting thioepoxide will be thiooxirane. The cyclic thiocarbonates prepared herein are typically of the following general formula:

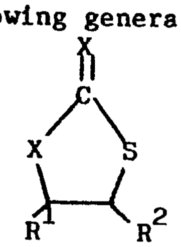

(II)

wherein the X component can each independently be sulphur or oxygen and wherein $R^1$ and $R^2$ taken separately are independently selected from the group consisting of hydrogen, aliphatic, alicyclic and aromatic radicals or $R^1$ and $R^2$ taken collectively is an alicyclic group.

The temperature at which the epoxide is reacted with carbon disulphide is in the range from 0° to 100°C and is preferably from 20° to 80°C. Atmospheric or superatmosphere pressures can be used but the pressure typically ranges from 0 to 10 bar.

By the term "amidine" is meant a compound containing the grouping:

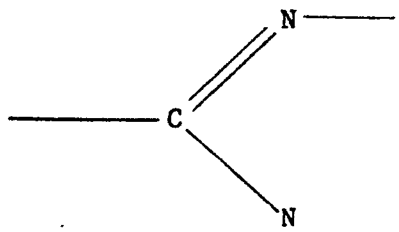

Conveniently the free valencies on the nitrogen atom are attached to carbon atoms or hydrogen atoms and the free valency on the carbon to another carbon or nitrogen atom. In the last mentioned case where the carbon atom is attached to a nitrogen atom, the structure comprises a guandine grouping and a compound containing this grouping is a guanidine.

A preferred class of amidines is the cyclic amidines. Cyclic amidines are defined as those amidines wherein at least one of the nitrogen atoms is part of an alicyclic or heterocyclic substituted or unsubstituted hydrocarbyl ring. In the case where the amidine is a guanidine then any two of the three nitrogen atoms may be in the same or different rings. Those nitrogen atoms which are not part of any such rings may form part of a substituted or unsubstituted hydrocarbyl group.

A preferred class of cyclic amidines is that in which the amidine group can form part of a fused ring system containing 6- and 5-membered rings or 6- and 7-membered rings or two six-membered rings, as for example in 1,5-diazabicyclo[4.3.0] non-5-ene, 1,8-diazabicyclo[5.4.0]undec-7-ene, or 1,5,7-triazabicyclo[4.4.0]dec-5-ene.

The amidine can be supported, that is chemically and physically bonded, to an inert solid and then added to the reaction mixture. In the supported form of the catalyst the surface atoms of the solid are bonded to one or more of the free valencies of the amidine or

guanidine group either directly or through an intermediate hydrocarbyl radical. In the case of cyclic amidines or guanidines the hydrocarbyl radical may constitute part of the ring structure of the molecule. The amidine is bonded to the support by conventional techniques known to those skilled in the art.

The inert solid may be either organic, for example a polymer, copolymer, resin and the like or it may be inorganic such as a silica, aluminosilicate, alumina, diatomaceous earth, zeolite, clay and the like.

The present invention is illustrated with reference to the following Examples and Comparative Example (not performed in accordance to the present invention). However, the Examples should not be construed as limiting the scope of this invention which includes equivalent modifications, variations and embodiments.

Example 1

A 50 ml round bottom flask fitted with a reflux condenser was charged with 7.2g of butene oxide, 7.6g of carbon disulphide, 5g of methanol and 0.1g of 1,5,7-triazabicyclo[4.4.0]dec-5-ene(TBD). The resulting solution was heated to 40°C and maintained at this temperature for 2 hours before cooling to room temperature. Analysis of the liquid product by gas chromatography showed a quantitative conversion of butene oxide with an 82% selectivity to butylene sulphide and a 10% selectivity to butylene trithiocarbonate. Small amounts of $(MeO)_2C=S$ and butylene dithiocarbonate were also found. No polymeric product was observed.

Comparative Example A

Example 1 was repeated in the absence of TBD. No reaction occured.

Comparative Example B

Example 1 was repeated except that 0.1g of triethylamine was used in place of TBD. Analysis of the liquid product showed a quantitative conversion of butene oxide with a 72% selectivity to butylene sulphide and a 6% selectivity to butylene thiocarbonate.

This Example, when compared with Example 1, carried out under identical conditions, demonstrates that higher selectivities to

desirable products are obtained.

Example 2

Example 1 was repeated except that 5g of butanethiol were used in place of methanol. Analysis of the liquid product showed a quantitative conversion of butylene oxide with a 49% selectivity to butylene dithiocarbonate and a 41% selectivity to butylene trithiocarbonate. Small amounts of $(BuS)_2C{=}S$ and $BuSCH_2CH(Et)SH$ were also formed.

Claims:

1 A process for the production of a sulphur-containing cyclic compound comprising a thioepoxide and/or a cyclic thiocarbonate which process comprises reacting carbon disulphide at a temperature in the range from 0 to 100°C with an epoxide in the presence as catalyst of an amidine.

2 A process according to claim 1 wherein the epoxide contains at least one moiety of the formula:

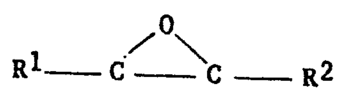

(I)

wherein $R^1$ and $R^2$ taken separately are independently selected from the group consisting of hydrogen, aliphatic, alicyclic and aromatic radicals or $R^1$ and $R^2$ taken collectively is an alicyclic group and has from 2 to 12 carbon atoms.

3 A process according to claim 2 wherein the epoxide is either ethylene oxide, propylene oxide, butylene oxide or a phenyloxirane.

4 A process according to any one of the preceding claims wherein the process is conducted in the liquid phase in the presence of a solvent.

5 A process according to claim 4 wherein the solvent is either a $C_1$ to $C_{12}$ aliphatic alcohol or a $C_1$ to $C_{12}$ aliphatic thiol.

6 A process according to claim 4 wherein the solvent is either methanol or ethanol.

7 A process according to any one of the preceding claims wherein the amidine is a cyclic amidine.

8 A process according to claim 7 wherein the amidine is a guanidine.

9 A process according to claim 7 wherein the cyclic amidine is one in which the amidine group forms part of a fused ring system containing either 6- and 5-membered rings, 6- and 7-membered rings or two six-membered rings.

10 A process according to any one of the preceding claims wherein the amidine is 1,5-diazabicyclo[4.3.0]non-5-ene; 1,8-diazabicyclo[5.4.0]undec-7-ene or 1,5,7-triazabicyclo[4.4.0]dec-5-ene.

European Patent Office

# EUROPEAN SEARCH REPORT

0207669
Application number

EP 86304544.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US - A - 3 457 277 (OSBORN, St. W. et al.)<br>* Claim 1 *<br>-- | 1 | C 07 D 331/02<br>C 07 D 331/04<br>C 07 D 333/00<br>C 07 D 335/02<br>C 07 D 337/00<br>C 07 D 339/06<br>C 07 D 327/04 |
| D,A | US - A - 3 124 596 (STANSBURY, H. A.; et al.)<br>* Claims *<br>---- | 1 | |

| TECHNICAL FIELDS SEARCHED (Int. Cl.4) |
|---|
| C 07 D 331/00<br>C 07 D 333/00<br>C 07 D 335/00<br>C 07 D 337/00<br>C 07 D 339/00<br>C 07 D 327/00 |

The present search report has been drawn up for all claims

| Place of search | Date of completion of the search | Examiner |
|---|---|---|
| VIENNA | 22-09-1986 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document